# FASCICULE DE BREVET EUROPEEN

(11) **EP 0 441 685 B1**
(45) Date de publication et mention de la délivrance du brevet: **15.09.1993**
(21) Numéro de dépôt: 91400229.0
(22) Date de dépôt: 31.01.1991
(51) Int. Cl.: B01J 27/18, B01J 27/185, C07C 51/377, C07C 57/03

(54) **Système catalytique et son application à l'oxydéshydrogénation d'acides carboxyliques saturés**
Katalytisches System und seine Verwendung bei der Oxydehydrierung von gesättigten Karbonsäuren
Catalytic system and its use in the oxydehydrogenation of saturated carboxylic acids

(30) Priorité: 07.02.1990 FR 9001391
(43) Date de publication de la demande: 14.08.1991
(73) Titulaire: ELF ATOCHEM S.A., 92091 Paris Cédex 42 (FR)
(72) Inventeur: Hecquet, Gérard, F-62400 Béthune (FR); Huchette, Dominique, F-62400 Locon (FR)

(56) Documents cités:
- EP-A- 0 263 005
- EP-A- 0 325 213
- CA-A- 1 186 673
- FR-A- 2 245 604
- GB-A- 2 090 589
- US-A- 3 809 727
- US-A- 4 427 792

## Description

La présente invention porte sur un système catalytique à base d'oxyde de fer et de phosphore, en vue de l'oxydéshydrogénation d'acides carboxyliques saturés en acides insaturés correspondants, en particulier en vue de l'oxydéshydrogénation de l'acide isobutyrique en acide méthacrylique.

On connaît, par les brevets US-A-4 364 856 et US-A-4 473 707, un catalyseur à base d'oxyde de fer et de phosphore, qui se présente sous une forme enrobée sur un support. Un tel catalyseur est désigné ci-après par l'expression «catalyseur enrobé». Son procédé de préparation consiste à mouiller partiellement un support, tel que la silice, avec une solution ou suspension colloïdale de SiO₂ dans l'eau ; mettre le support partiellement mouillé en contact avec une poudre du catalyseur ; et agiter le mélange ainsi réalisé pour former le catalyseur enrobé, lequel est ensuite séché, puis calciné.

Le catalyseur proprement dit est représenté par la formule empirique AₐFe_{b}P_{c}D_{d}O_{x,} où :
- A est choisi parmi Al, B, Be, Cd, Co, Cr, Ga, Ge, In, Ni, Te, Th, Ti, Tl, U, V, Zn, Zr, les terres rares et leurs mélanges ;
- D est choisi parmi Ag, Cu, Mn et leurs mélanges ; et
- a = 0-1,0 ; b = 0,75-1,5 ; c = 1,0-2,0 ; d = 0-2,0 ; a + d est supérieur à 0; et x est le nombre d'oxygène nécessaire pour satisfaire les exigences de valence des éléments restants.

Un tel catalyseur enrobé donne satisfaction des points de vue de la sélectivité et de la conversion. Toutefois, il possède l'inconvénient de ne pas présenter de résistance mécanique élevée ; autrement dit, dans le cas où la réaction d'oxydéshydrogénation est conduite dans un réacteur industriel tubulaire de grande hauteur, le catalyseur risque de se détériorer lors du remplissage des tubes. De plus, la réaction ayant lieu en phase gazeuse, il a également tendance à se détériorer au cours du temps, sous l'action de la pression du flux gazeux, ce qui a pour conséquence d'augmenter la perte de charge. En outre, si l'enrobage par la phase catalytique active est en trop forte couche, les pores seront trop petits pour que l'acide formé s'en désorbe. Il faudra donc, dans ce cas, prévoir des grains de petite dimension, ce qui augmentera la perte de charge.

On connaît également ces mêmes catalyseurs à base d'oxyde de fer et de phosphore, qui se présentent sous une forme dite massique. Pour les préparer, on peut procéder de la façon suivante :

On dissout, dans un solvant tel que l'eau, la quantité désirée d'un composé contenant du fer. On y incorpore la quantité appropriée de phosphore sous la forme d'un acide ou d'une solution de sel dissous. De la silice colloïdale peut également être ajoutée jusqu'à 15% en poids par rapport au mélange résultant afin de donner au catalyseur la résistance physique souhaitée. Le pH de la solution est ensuite ajusté à 7 par l'addition d'une base, ce qui conduit à la formation d'un précipité de catalyseur brut fer/phosphate qui est séché après lavage, après combinaison avec des sels de métaux alcalins ou alcalino-terreux, s'il est prévu que le catalyseur comporte en outre de tels métaux. En variante, on peut ajouter les sels de métaux alcalins ou alcalino-terreux à la solution de sel de fer et de phosphore avant la neutralisation. Après séchage, le catalyseur est broyé à la finesse désirée, puis calciné.

Avec de tels catalyseurs, la conversion de l'acide isobutyrique est de l'ordre de 90%, et la sélectivité en acide méthacrylique de l'ordre de 80%. Cependant, ces catalyseurs présentent l'inconvénient de ne pouvoir être utilisés qu'en petits grains. Par conséquent, l'utilisation de ces catalyseurs dans un tube en lit fixe avec une grande hauteur de catalyseur ne serait pas possible, car l'augmentation de pression serait trop élevée et, par conséquent, la sélectivité en acide formé diminuerait.

On connaît également, par le brevet CA-A-1 186 673, un système catalytique à deux composants, comprenant, en mélange physique, un catalyseur du type précité fer/phosphate, et un support inerte (silice) dopé au phosphate, ledit support étant préparé par formation d'une bouillie aqueuse d'un support inerte et d'acide phosphorique, élimination du solvant par évaporation pour former une masse séchée, et calcination de la masse séchée. Un tel système catalytique est utile notamment pour la fabrication d'acide méthacrylique suivant laquelle l'acide isobutyrique et l'oxygène sont placés en contact physique avec lui. Toutefois, il présente les mêmes inconvénients que les catalyseurs dits massiques décrits ci-dessus.

La demande de brevet français FR-A-2 245 604 décrit un procédé de préparation d'acides α,β-insaturés par oxydation en phase gazeuse des acides saturés, en présence d'un catalyseur contenant du fer, du phosphore, au moins un élément des Groupes Ia et IIa de la Classification Périodique, et de l'oxygène, ce catalyseur pouvant être supporté sur de la silice microsphéroïdale.

La Société déposante a donc cherché à améliorer la fabrication des catalyseurs de type précité, en augmentant la dimension des grains de catalyseur pour éviter une augmentation de pression trop forte.

La Société Déposante a maintenant découvert que si l'on utilise un support macroporeux, imprégnable à coeur, on obtient des catalyseurs qui présentent de très bonnes performances catalytiques, qui sont mécaniquement plus robustes que les catalyseurs massiques ou enrobés, et qui contrairement à ces derniers, ne s'écrasent pas au chargement d'un réacteur tubulaire de grande hauteur et ne s'effritent pas sous l'effet de la pression (flux gazeux) lors du fonctionnement du procédé, d'où il résulte que la perte de charge dans le réacteur restera constante. La présente invention, basée sur cette découverte, apporte donc une solution aux problèmes posés sans nuire au compromis sélectivité/conversion.

La présente invention a donc d'abord pour objet un catalyseur de formule générale FePₓMe_{y}O_{z}, dans laquelle :
- Me représente au moins l'un des éléments suivants : Li, Na, K, Rb, Cs, Mg, Ca, Sr et Ba ;
- x vaut de 0,2 à 3,0 ;
- y vaut de 0,1 à 2,0 ; et
- z est la quantité d'oxygène liée aux autres éléments et correspondant à leur état d'oxydation,

ledit catalyseur étant associé à un support, caractérisé par le fait que ledit support est un support macroporeux, imprégnable à coeur, présentant une surface spécifique inférieure ou égale 1 m²/g, un volume de pores compris entre 0,2 et 1 cm³/g et un diamètre moyen de pore supérieur ou égal à 1 µm, et que la matière active est déposée à la surface de tous les pores dudit support, ledit catalyseur se présentant sous la forme de grains de support imprégnés de matière active, qui ont une dimension comprise entre 0,5 et 10 mm.

On choisit notamment un catalyseur pour lequel le rapport atomique P/Fe est supérieur à 1, et qui satisfait la relation : 0,01<Me/Fe<1,0.

De préférence, le support présente une surface spécifique inférieure à environ 0,3 m²/g, étant par exemple de l'ordre de 0,1 m²/g ; un volume de pores compris entre environ 0,35 et 0,4 cm³/g ; et un diamètre moyen de pore supérieur ou égal à 1 µm ; en particulier, on peut choisir un support dont environ 70 à 90% des pores ont un diamètre supérieur à 10 µm.

Ainsi, le choix d'un support de dimension de pores suffisamment grande permet que l'acide formé se désorbe facilement, ce qui n'est pas le cas avec les catalyseurs massiques, nécessairement en petits grains, de même qu'avec les catalyseurs enrobés, dans lesquels les pores de la couche catalytique extérieure sont également trop petits. Dans le cas de ces catalyseurs connus, les molécules d'acide de départ ont accès aux sites actifs se trouvant dans les pores, mais l'acide formé se désorbe avec difficulté, et, s'il est désorbé, il se dégrade. Par ailleurs, le fait de pouvoir, selon l'invention, utiliser des grains de support relativement gros, évite les pertes de charge.

Le support macroporeux de catalyseur selon l'invention est choisi notamment parmi la silice, l'alumine, le carbure de silicium, l'oxyde de titane, la zircone et leurs mélanges.

Le système catalytique selon l'invention se présente avantageusement sous la forme de grains de support imprégnés de matière active, qui ont une dimension comprise entre environ 0,8 et 1,6 mm. Il contient notamment environ 2 à 50% en poids de matière active.

Un tel système catalytique peut être préparé par trempage du support macropreux dans une solution aqueuse d'acide phosphorique contenant un composé du fer (tel que Fe (NO₃)₃. 9H₂O) et un composé du métal Me, égouttage, séchage du support ainsi imprégné, et calcination.

Le trempage peut durer de quelques secondes à quelques heures. Toutefois, le temps de trempage n'est pas critique. Le séchage et la calcination sont des étapes bien connues de l'homme du métier. A titre d'exemples, on peut mentionner un séchage à 120°C environ pendant 16-24 heures environ, ou à 180°C environ pendant 4-7 heures environ, et une calcination à environ 400-500°C, pendant environ 6-24 heures.

La présente invention a également pour objet un procédé d'oxydéshydrogénation d'acides carboxyliques saturés pour former des acides carboxyliques alpha, bêta insaturés, ce procédé consistant à mettre en contact lesdits acides insaturés avec de l'oxygène moléculaire ou un gaz contenant de l'oxygène, et, le cas échéant, de la vapeur ou un gaz diluant inerte, en phase vapeur, à une température de réaction d'environ 250 à 600°C, en présence d'un catalyseur sur support contenant des oxydes de fer et de phosphore, ce catalyseur étant tel que défini ci-dessus.

Les exemples suivants illustrent l'invention sans toutefois en limiter la portée. Dans ces exemples, les pourcentages sont donnés en poids, sauf indication contraire.

### Exemple 1: Préparation d'un catalyseur imprégné selon l'invention, de composition FeP_{1,23}Cs_{0,15}

On prépare à froid une solution de CsNO₃, de Fe(NO₃)₃. 9H₂O, dans H₃PO₄ à 85% (couleur lie-de-vin), en employant les quantités nécessaires. On trempe, pendant quelques heures, dans la solution obtenue, un panier renfermant de la silice de surface spécifique inférieure à 1 m²/g de volume de pores Vₚ tel que 0,35 ≦ vₚ ≦ 0,8 cm³/g, 15 à 25% des pores ayant un diamètre inférieur à 1 micron et 4 à 6% des pores ayant un diamètre inférieur à 0,1 micron. Cette silice étant macroporeuse, imprégnable à coeur, tous ses pores se remplissant alors de la solution.

Après égouttage, on sèche la silice imprégnée contenue en couches minces dans des plateaux à 120°C pendant une nuit, puis on calcine à 460°C pendant 16 heures.

On obtient des grains imprégnés de dimensions d'environ 4,8 mm x 4,8 mm, qui sont à environ 30% de matière catalytiquement active. La composition d'ensemble est celle définie ci-dessus.

### Exemple 2 (comparatif) : Préparation d'un catalyseur massique, non-supporté, de composition FeP_{1,23}Cs_{0,15}

On met en solution dans H₃PO₄ concentré (85%), CsNO₃, puis Fe(NO₃)₃·9H₂O (solution lie-de-vin), en employant les quantités nécessaires. Puis, on ajoute 3% de silice colloïdale par rapport au poids total de la solution.

On conduit une évaporation, puis un séchage à 120°C pendant 17 heures et à 180°C pendant 4 heures. Ensuite, on effectue un broyage, puis on mélange, et on effectue un pastillage, pour obtenir des pastilles d'environ 5 mm de diamètre et de hauteur h telle que 3,5≦h≦4,5 mm, puis concassage pour obtenir des grains de diamètre d tel que 0,8≦d≦1,6 mm, et enfin une calcination à 460°C, pendant 16 heures.

Les caractéristiques de ce catalyseur sont les suivantes :
- surface spécifique A tell que 2≦A≦6 m²/g ;
- volume des pores Vₚ tel que 0,17≦Vₚ≦0,21 cm³/g ;
- 63 à 85% des pores ont un diamètre inférieur à 1 µm.
- 17 à 21% des pores ont un diamètre inférieur à 0,1 µm.

### Exemple 3 : Obtention d'acide méthacrylique par oxydéshydrogénation de l'acide isobutyrique

On introduit dans une colonne de 14 mm de diamètre interne, 15 ml du catalyseur de l'Exemple 1 sur une hauteur de 8,5 cm. Il se forme très peu de poussière lors de ce chargement, le catalyseur présentant une forte résistance à l'écrasement. La colonne est chauffée par un bain de sel.

Les conditions de la réaction sont les suivantes :
- Température du bain de sel : 440°C
- Temps de séjour : 1,4 seconde
- H₂O/acide isobutyrique : 12
- O₂/acide isobutyrique : 0,75
- Acide isobutyrique dans le gaz : 6%

La conversion de l'acide isobutyrique est de 90%, et de sélectivité en acide méthacrylique est de 84%.

### Exemple 4 (comparatif)

On a procédé comme dans l'Exemple 3, mais en utilisant le catalyseur de l'Exemple 2, à raison de 1 volume de catalyseur/2 volumes de quartz, avec une température au point chaud de 415°C, un temps de séjour de 0,8 seconde, un rapport O₂/acide isobutyrique de 1, les autres conditions étant identiques.

On a observé une forte production de poussière au chargement, et un accroissement de la perte de charge au cours du temps, bien que la conversion initiale ait été de 90% et la sélectivité en acide méthacrylique, de 80%.

## Revendications

1. Catalyseur de formule générale FePₓMe_{y}O_{z}, dans laquelle :
- Me représente au moins l'un des éléments suivants : Li, Na, K, Rb, Cs, Mg, Ca, Sr et Ba ;
- x vaut de 0,2 à 3,0 ;
- y vaut de 0,01 à 2,0 ; et
- z est le quantité d'oxygène liée aux autres éléments et correspondant à leur état d'oxydation,
ledit catalyseur étant associé à un support, caractérisé par le fait que ledit support est un support macroporeux, imprégnable à coeur, présentant une surface spécifique inférieure ou égale 1 m²/g, un volume de pores compris entre 0,2 et 1 cm³/g et un diamètre moyen de pore supérieur ou égal à 1 µm, et que la matière active est déposée à la surface de tous les pores dudit support, ledit catalyseur se présentant sous la forme de grains de support imprégnés de matière active, qui ont une dimension comprise entre 0,5 et 10 mm.

2. Catalyseur selon le revendication 1, caractérisé par le fait que le support est choisi parmi la silice, l'alumine, le carbure de silicium, l'oxyde de titane, la zircone et leurs mélanges.

3. Catalyseur selon l'une des revendications 1 et 2, caractérisé par le fait qu'il contient de 2 à 50% en poids de matière active.

4. Catalyseur selon l'une des revendications 1 à 3, caractérisé par le fait qu'il a été préparé par trempage du support macroporeux dans une solution aqueuse d'acide phosphorique contenant un composé de fer et un composé du métal Me, égouttage, séchage du support ainsi imprégné, et calcination.

5. Procédé d'oxydéshydrogénation d'acides carboxyliques saturés pour former des acides carboxyliques alpha, bêta insaturés, ce procédé consistant à mettre en contact lesdits acides insaturés avec de l'oxygène moléculaire ou un gaz contenant de l'oxygène, et, le cas échéant, de la vapeur ou un gaz diluant inerte, en phase vapeur, à une température de réaction d'environ 250 à 600°C, en présence d'un catalyseur de formule générale FePₓMe_{y}O_{z}, dans laquelle :
- Me représente au moins l'un des éléments suivants : Li, Na, K, Rb, Cs, Mg, Ca, Sr et Ba ;
- x vaut de 0,2 à 3,0 ;
- y vaut de 0,01 à 2,0 ; et
- z est la quantité d'oxygène liée aux autres éléments et correspondant à leur état d'oxydation,
ledit catalyseur étant associé à un support, caractérisé par le fait que ledit support est un support macroporeux, imprégnable à coeur, présentant une surface spécifique inférieure ou égale 1 m²/g, un volume de pores compris entre 0,2 et 1 cm³/g et un diamètre moyen de pore supérieur ou égal à 1 µm, et que la matière active est déposée à la surface de tous les pores dudit support, ledit catalyseur se présentant sous la forme de grains de support imprégnés de matière active, qui ont une dimension comprise entre 0,5 et 10 mm.

6. Procédé selon la revendication 5, caractérisé par le fait que le support du catalyseur est choisi parmi la silice, l'alumine, le carbure de silicium, l'oxyde de titane, la zircone et leurs mélanges.

7. Procédé selon l'une des revendications 5 et 6, caractérisé par le fait que le catalyseur contient de 2 à 50% en poids de matière active.

8. Procédé selon l'une des revendications 5 à 7, caractérisé par le fait que le catalyseur a été préparé par trempage du support macroporeux dans une solution aqueuse d'acide phosphorique contenant un composé du fer et un composé du métal Me, égouttage, séchage du support ainsi imprégné, et calcination.

9. Procédé selon l'une des revendications 5 à 8, caractérisé par le fait que l'acide carboxylique saturé de départ est l'acide isobutyrique.

## Claims

1. Catalyst of the general formula FePₓMe_{y}O_{z}, in which:
- Me denotes at least one of the following elements: Li, Na, K, Rb, Cs, Mg, Ca, Sr and Ba;
- x has the value of 0.2 to 3.0;
- y has the value of 0.01 to 2.0; and
- z is the quantity of oxygen bonded to the other elements and corresponding to their oxidation state,
the said catalyst being coupled with a support, characterised in that the said support is a macroporous support capable of being impregnated throughout, having a specific surface lower than or equal to 1 m²/g, a pore volume of between 0.2 and 1 cm³/g and a mean pore diameter greater than or equal to 1 µm, and in that the active material is deposited on the surface of all the pores of the said support, the said catalyst being in the form of support particles impregnated with active material, which have a dimension of between 0.5 and 10 mm.

2. Catalyst according to Claim 1, characterised in that the support is chosen from silica, alumina, silicon carbide, titanium oxide, zirconia and mixtures thereof.

3. Catalyst according to either of Claims 1 and 2, characterised in that it contains from 2 to 50 % by weight of active material.

4. Catalyst according to one of Claims 1 to 3, characterised in that it has been prepared by soaking the macroporous support in an aqueous solution of phosphoric acid containing an iron compound and a compound of the metal Me, draining, drying the support impregnated in this way and calcining.

5. Process for oxidative dehydrogenation of saturated carboxylic acids to form alpha,beta-unsaturated carboxylic acids, this process consisting in bringing the said unsaturated acids into contact with molecular oxygen or a gas containing oxygen and, if appropriate, steam or an inert diluent gas, in vapour phase, at a reaction temperature of approximately 250 to 600°C, in the presence of a catalyst of the general formula FePₓMe_{y}O_{z}, in which:
- Me denotes at least one of the following elements: Li, Na, K, Rb, Cs, Mg, Ca, Sr and Ba;
- x has the value of 0.2 to 3.0;
- y has the value of 0.01 to 2.0; and
- z is the quantity of oxygen bonded to the other elements and corresponding to their oxidation state,
the said catalyst being coupled with a support, characterised in that the said support is a macroporous support capable of being impregnated throughout, having a specific surface lower than or equal to 1 m²/g, a pore volume of between 0.2 and 1 cm³/g and a mean pore diameter greater than or equal to 1 µm, and in that the active material is deposited on the surface of all the pores of the said support, the said catalyst being in the form of support particles impregnated with active material, which have a dimension of between 0.5 and 10 mm.

6. Process according to Claim 5, characterised in that the catalyst support is chosen from silica, alumina, silicon carbide, titanium oxide, zirconia and mixtures thereof.

7. Process according to either of Claims 5 and 6, characterised in that the catalyst contains from 2 to 50 % by weight of active material.

8. Process according to one of Claims 5 to 7, characterised in that the catalyst has been prepared by soaking the macroporous support in an aqueous solution of phosphoric acid containing an iron compound and a compound of the metal Me, draining, drying the support impregnated in this way and calcining.

9. Process according to one of Claims 5 to 8, characterised in that the starting saturated carboxylic acid is isobutyric acid.

## Patentansprüche

1. Katalysator der allgemeinen Formel FePₓMe_{y}O_{z}, worin
- Me mindestens eines der folgenden Elemente repräsentiert: Li, Na, K, Rb, Cs, Mg, Ca, Sr und Ba;
- x einen Wert von 0,2 bis 3,0 hat;
- y einen Wert von 0,01 bis 2,0 hat; und
- z die an andere Elemente gebundene Sauerstoffmenge ist und dem Oxidationszustand dieser Elemente entspricht,
wobei der genannte Katalysator einem Träger zugeordnet ist, dadurch gekennzeichnet, daß der genannte Träger ein makroporöser, durch und durch imprägnierbarer Träger ist, der eine spezifische Oberfläche von weniger als oder gleich 1 m²/g, ein Porenvolumen zwischen 0,2 und 1 cm³/g und einen mittleren Porendurchmesser von mehr als oder gleich 1 µm aufweist, und daß der aktive Stoff auf der Oberfläche aller Poren des genannten Trägers abgelagert ist, wobei der Katalysator in Form von mit aktivem Stoff imprägnierten Trägerkörnern vorliegt, die eine Abmessung zwischen 0,5 und 10 mm besitzen.

2. Katalysator nach Anspruch 1, dadurch gekennzeichnet, daß der Träger ausgewählt ist aus der Gruppe bestehend aus Kieselerde, Tonerde, Siliziumkarbid, Titanoxid, Zirkonerde und deren Mischungen.

3. Katalysator nach Anspruch 1 oder 2, dadurch gekennzeichnet, daß er 2 bis 50 Gew.-% aktiven Stoff enthält.

4. Katalysator nach einem der Ansprüche 1 bis 3, dadurch gekennzeichnet, daß er durch Eintauchen des makroporösen Trägers in eine eine Eisenverbindung und eine Verbindung des Metalls Me enthaltende wäßrige Phosphorsäurelösung, Abtropfenlassen und Trocknen des so imprägnierten Trägers sowie Kalzinieren hergestellt worden ist.

5. Verfahren zur Oxydehydrierung von gesättigten Carbonsäuren zwecks Bildung von α-, β-ungesättigten Carbonsäuren, welches Verfahren darin besteht, die genannten ungesättigten Säuren mit Molekularsauerstoff oder einem sauerstoffhaltigen Gas und gegebenenfalls mit Dampf oder einem inerten Verdünnungsgas, in Dampfphase bei einer Reaktionstemperatur von etwa 250° bis 600°C in Gegenwart eines Katalysators der allgemeinen Formel FePₓMe_{y}O_{z} in Kontakt zu bringen, in welcher Formel
- Me mindestens eines der folgenden Elemente repräsentiert: Li, Na, K, Rb, Cs, Mg, Ca, Sr und Ba;
- x einen Wert von 0,2 bis 3,0 hat;
- y einen Wert von 0,01 bis 2,0 hat; und
- z die an andere Elemente gebundene Sauerstoffmenge ist und dem Oxidationszustand dieser Elemente entspricht,
wobei der genannte Katalysator einem Träger zugeordnet ist, dadurch gekennzeichnet, daß der genannte Träger ein makroporöser, durch und durch imprägnierbarer Träger ist, der eine spezifische Oberfläche von weniger als oder gleich 1 m²/g, ein Porenvolumen zwischen 0,2 und 1 cm³/g und einen mittleren Porendurchmesser von mehr als oder gleich 1 µm aufweist, und daß der aktive Stoff auf der Oberfläche aller Poren des genannten Trägers abgelagert ist, wobei der Katalysator in Form von mit aktivem Stoff imprägnierten Trägerkörnern vorliegt, die eine Abmessung zwischen 0,5 und 10 mm besitzen.

6. Verfahren nach Anspruch 5, dadurch gekennzeichnet, daß der Träger des Katalysators ausgewählt ist aus der Gruppe bestehend aus Kieselerde, Tonerde, Siliziumkarbid, Titanoxid, Zirkonerde und deren Mischungen.

7. Verfahren nach Anspruch 5 oder 6, dadurch gekennzeichnet, daß der Katalysator 2 bis 50 Gew.-% aktiven Stoff enthält.

8. Verfahren nach einem der Ansprüche 5 bis 7, dadurch gekennzeichnet, daß der Katalysator durch Eintauchen des makroporösen Trägers in eine eine Eisenverbindung und eine Verbindung des Metalls Me enthaltende wäßrige Phosphorsäurelösung, Abtropfenlassen und Trocknen des so imprägnierten Trägers sowie Kalzinieren hergestellt worden ist.

9. Verfahren nach einem der Ansprüche 5 bis 8, dadurch gekennzeichnet, daß die gesättigte Ausgangs-Carbonsäure Isobuttersäure ist.
